# EUROPEAN PATENT SPECIFICATION

(11) **EP 4 510 154 B1**
(45) Date of publication and mention of the grant of the patent: **28.01.2026**
(21) Application number: 24194931.2
(22) Date of filing: 16.08.2024
(51) Int. Cl.: H01F 41/02, H01F 41/04, A61B 5/06, A61B 34/20, B33Y 80/00

(54) **A SYSTEM AND METHOD OF SENSING CATHETER'S LOCATION AND FORCE**
SYSTEM UND VERFAHREN ZUR ERFASSUNG DER POSITION UND KRAFT EINES KATHETERS
SYSTÈME ET PROCÉDÉ DE DÉTECTION DE LA POSITION ET DE LA FORCE D'UN CATHÉTER

(30) Priority: 17.08.2023 US 202363533258 P
(43) Date of publication of application: 19.02.2025
(73) Proprietor: Biosense Webster (Israel) Ltd., 2066717 Yokneam (IL)
(72) Inventor: HAYAT, Nathanel Yehuda David, 2066717 Yokneam (IL); BREEN, Barry Neal, 2066717 Yokneam (IL); ZOHAR, Daniel, 2066717 Yokneam (IL); COHEN, Avraham, 2066717 Yokneam (IL)
(74) Representative: Carpmaels & Ransford LLP

(56) References cited:
- CN-B- 105 632 893
- US-A1- 2016 358 897
- CANADA JORGE ET AL: "Monolithically 3D-Printed, Miniature Solenoids with Soft Magnetic Core for Compact Systems", 2023 13TH INTERNATIONAL CONFERENCE ON POWER, ENERGY AND ELECTRICAL ENGINEERING (CPEEE), IEEE, 25 February 2023 (2023-02-25), pages 116 - 120, XP034405952, [retrieved on 20230823], DOI: 10.1109/CPEEE56777.2023.10217558
- DASSAULT SYSTEMS: "The Best Resins for 3D Printing", 1 January 2025 (2025-01-01), XP093332887, Retrieved from the Internet <URL:https://www.3ds.com/make/solutions/blog/best-resins-3d-printing>

## Description

### CROSS-REFERENCE TO RELATED APPLICATION

This application claims priority of U.S. Patent Provisional Application 63/533,258, filed on August 17, 2023.

### TECHNOLOGICAL FIELD

The present invention is in the field of catheters and particularly relates to systems and methods for sensing catheter's location and the force applied thereby.

### BACKGROUND

In various therapeutic and diagnostic procedures, a catheter/probe is inserted into a patient's body (e.g., chamber of the heart) and to be brought into contact with a body tissue there. Typically, in such procedures, it is necessary to determine the catheter's location within the body (i.e., the location at which a distal tip of the catheter engages the body tissue) as well as the pressure applied thereby to the tissue.

Catheters having integrated location and pressure sensors for sensing the location of the catheter and the pressure/force applied thereby at the contact region with the tissue, are generally known. Such catheters typically utilize inductive coils for determining the location of the catheter within the body and/or the pressure/force applied thereby to a body tissue it engages with.

Conventional techniques for such catheters often utilize wire-winded coils to which a ferrite core may be manually inserted after the winding. The coils fabricated in this way are then soldered to a cable that carries the coil signal to processing circuits, and fitted within the catheter's body.

The disclosure of Canada Jorge et al: "Monolithically 3D-printed, Miniature Solenoids with Soft Magnetic Core for Compact Systems", 2023 13th International Conference on Power, Energy and Electrical Engineering (CPEEE), IEEE, 25 February 2023, pages 116-120, XP034405952, DOI: 10.1109/CPEEE56777.2023.10217558 provides monolithically 3D-printed, three-dimensional, three-material, cored inductors for use in compact electromagnetic systems. The 3D-printed inductors are made via material extrusion using three PLA-based materials (dielectric, conductive, and soft magnetic).

The disclosure of CN105632893B provides a three-dimensional (3D) printing based method for producing a micro-inductor, belonging to the technical field of production of the micro-inductor. The micro-inductor prepared through printing is a three-dimensional inductor or a planar micro-inductor.

The disclosure of US2016/358897A1 provides a method including forming at least one passive structure on a substrate by a build-up process; introducing one or more integrated circuit chips on the substrate; and introducing a molding compound on the at least one passive structure and the one or more integrated circuit chips.

### SUMMARY OF THE INVENTION

The invention is defined in the independent claims. Further embodiments are provided in the dependent claims.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Fig. 1A** is a flow diagram of a method **100** according to an embodiment of the present invention for fabricating electrical components with one or more open magnetic circuit electric coils therein;
**Figs. 1B to 1D** are respectively a perspective view, several lateral cross-sectionals views and a side-view cross-section, of a model of an example electric component **210** fabricated by the technique of the present invention;
**Fig. 1E****,** is schematic perspective view illustration of two example electric components **210(a) 210(b)** of the present invention, which are suitable for use as medical instrument sensors;
**Figs. 1F** **and** **1G****,** are schematic perspective view illustration of six example electric components **210(c)** to **210(h)** according to the present invention, each including a single open magnetic circuit coil 3D-printed by the technique of the present invention; and
**Fig. 2** shows a side-view of a medical instrument **1000** such as a catheter according to an embodiment of the present invention, in which electric components **210** of the present invention are furnished as position and force sensors.

### DETAILED DESCRIPTION OF EMBODIMENTS

There is a need in the art of catheters, for physically agile and sensitive catheters having compact/narrow dimensions, that are capable of providing accurate feedback on the location of the catheter in the body, and the contact interface between the catheter and the tissue and the force (e.g., vector) applied between them.

U.S. patent No. U.S.8,535,308 and U.S. patent application No. U.S. 2020/015693, which are both assigned to the assignee of the present Application, disclose for example configurations of such catheters utilizing sensory circuits having several coils, for sensing the location and orientation of the catheter body, as well as the force applied thereby at the interface with the tissue.

One challenge in the art of such catheters is to fit accurate location and force sensors within the narrow body of the catheter, whose lateral dimensions are typically only several millimeters. Indeed, as indicated above, in conventional catheters technologies, the location and force sensors generally utilize miniature wire-winded coils to which a ferrite core may be manually inserted with an open magnetic circuit configuration in order to yield sufficient induced-voltages suitable for location and force sensing of the catheter components. These conventional techniques are however time consuming and costly and involve delicate manual operations for fitting of the coils within the small dimensions of the catheters body.

However, other conventional techniques for coil fabrication are also less suited for fabrication of high induced voltage miniature open magnetic circuit coils, and are lacking, for instance with their ability to fabricate miniature open magnetic circuit coils with longitudinal dimension to lateral dimension aspect ratio of at least 3 or 4 (e.g., coil longitude of about 2mm and lateral/width of about 0.6 mm). Accordingly, the conventional techniques are also lacking when it comes to fabrication of miniature open magnetic circuit coils having high sensitivity/induced-voltage (e.g., in the order of 0.1 to 1 µV/(Gauss*Hz) corresponding to 0.001 to 0.01 V/(T*Hz)) This is because the sensitivity/induced-voltage typically depends on the longitudinal dimension to lateral dimension aspect ratio (where too small aspect ratio significantly decreases the effective permeability of the magnetic core as it depends on the geometry). For instance, conventional sheet-lamination technologies (also known as green sheet lamination), by which inductor coils are fabricated by printing internal inductor electrodes on a plurality of magnetic or dielectric green sheets and stacking the sheets, are not reliable and prone to manufacturing defects when it comes to fabrication of miniature coils with dimensions as described above and/or having sufficient length to width ratio needed to yield the high induced voltage.

Therefore, there is a need in the art for more efficient, cost-effective techniques for fabrication of such catheters and for the fabrication of small high sensitivity coils (i.e., having high induced voltage in response to magnetic fields) suitable for use for location and or force sensing in catheters such as those disclosed above. Moreover, there is a need in the art for automated fabrication techniques of such coils, suitable for mass production with yield of standardized coils having less variability between fabricated coils of the same design. Further, in the interest of further advent in the art of catheters, there is also a need in the art to facilitate even smaller and/or of higher impedance/induced-voltage, so as to improve either or both of the sensitivity and agility, and/or reduced dimensions, of the catheter.

Therefore, the present invention provides a novel positioning sensory system and novel configurations and fabrication methods for electric components including open magnetic circuit coil(s) suited for magnetic sensors of high induced-voltage, for example for use as position sensors to be furnished within narrow dimensions of a catheter body.

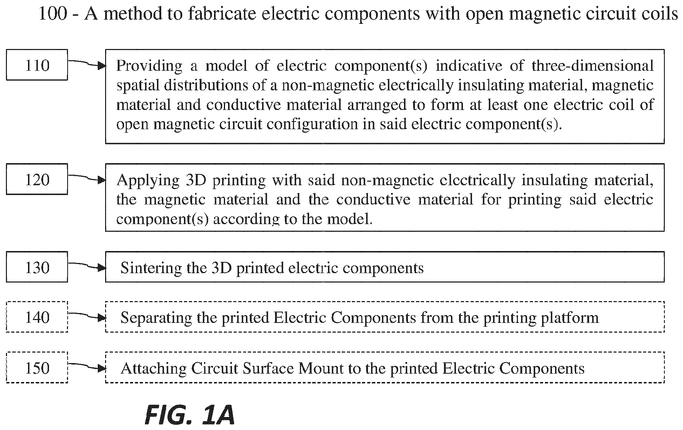

Reference is now made to **Fig. 1A** illustrating, in a flow diagram, a method **100** according to embodiments of the present invention, to fabricate electrical components with one or more electric coils therein having an open magnetic circuit configuration suitable for use in catheter's position sensor.

Advantageously, the method **100** utilizes a 3D-printing technology for printing electric components having ceramic and/or glass-ceramic solid main body (non-laminated bulk) with open magnetic circuit electric coils embedded therein. The open magnetic circuit electric coils embedded in the electric components are fabricated with at least three different materials which have different electromagnetic properties including: a magnetic material, a conductive material, and a non-magnetic dielectric (electrically insulating) material.

In this connection the phrase *open magnetic circuit configuration* in relation to the coils fabricated by the technique of the present invention should be understood herein as relating to coil structures that pose substantially no shielding of external magnetic fields (i.e., enabling the external magnetic field pass through the coil core without substantially bypassing around the core). The terms *magnetic- material* and/or *particles* is used herein to designate materials of high relative magnetic permeability µᵣ, for example having relative permeability preferably of µᵣ ≥ 100 relative to the vacuum permeability µ₀. Conversely the terms *non-magnetic- material* and or particles are used herein to designate materials of low relative permeability µᵣ which is substantially lower than that of the magnetic materials used in the electronic components' fabrication and typically in the order of µᵣ ~ 1.

Moreover, it should be noted that the phrases multi-material three-dimensional-printing (i.e. here in after also referred to for short as *3D-printing*) are used herein to designate multi-material *3D-printing* techniques in which material adhesion, polymerization and/or curing are carried out layer by layer while enabling selective pixelated deposition, adhesion, polymerization and/or curing of voxels of several different materials (several distribution of different material voxels) in each layer. To this end, the *3D-printing* (*multi-material*) technique (used in the present application should not be confused with other additive manufacturing techniques such as sheet-lamination techniques (e.g. green-sheet lamination), material extrusion or others, which do not facilitate selective pixelated deposition, adhesion, polymerization and/or curing of voxels of different materials within the same layer (a feature which is also referred to herein as *in-layer* pixelation).

Thus, in operation **110** of method **100,** a model of the one or more electric components **210** which are to be 3D-printed is provided. The model is indicative 3D spatial distribution (voxel maps) of at least three materials, **221, 222,** and **223,** which are to be 3D-printed by multi-material 3D-printing in order to form the electrical components of the present invention. Particularly the model is indicative of the respective distribution of the three materials **221, 222** and **223** according to which one or more open-magnetic circuit coils (typically of miniature dimension) are to be formed in the one or more electronic components **210** printed by the multi-material 3D-printing.

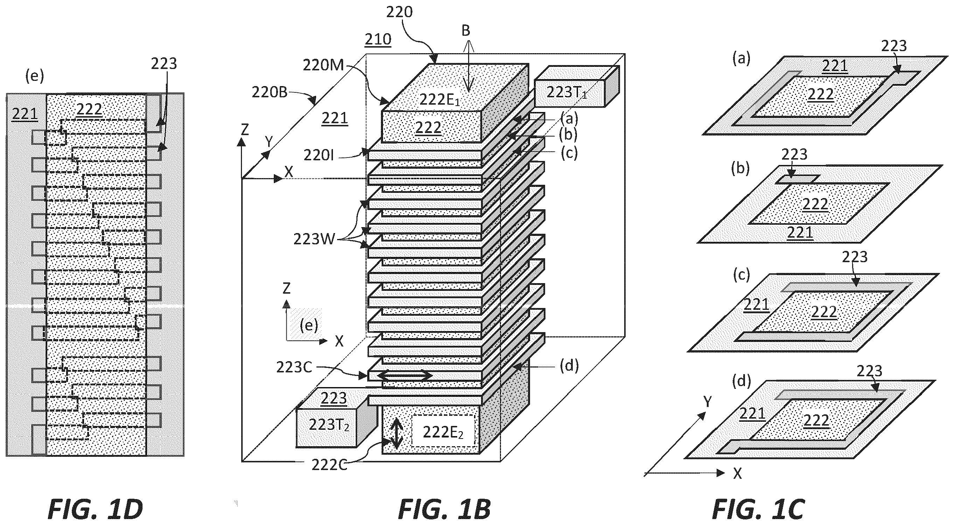

With reference to **Fig. 1B** a perspective view of a model of one electric component **210** which includes in this non-limiting example a single open magnetic circuit coil **220** is schematically illustrated. As shown the model is indicative of a 3D-distribution of conductive material **223** forming at least one electric channel **223C** of the coil **220** extending between at least two end terminals **223T₁** and **223T₂** thereof which are typically formed by the same conductive material **223.** Additionally, the model **220** is indicative of a 3D-distribution of magnetic material **222** forming at least one magnetic channel **222C** extending between at least two distal-end facets thereof **222E₁** and **222E₂.** Typically, the two distal-end facets **222E₁** and **222E₂** of the magnetic channel **222C** present the input/output of magnetic flux flow through the magnetic channel **222C** and accordingly the general direction/axis of sensitivity of the coil to external magnetic field **B** may typically be considered as the direction spanned between these distal-end facets **222E₁** and **222E₂** , as shown by the double arrow of **B** in the figures (e.g. in some implementations regardless of the actual shape of the magnetic channel 3D printed between them).

The material distributions **222** and **223** forming the magnetic channel **222C** and the electric channel **223C** of the modeled coil **220,** are configured in the model **220** with coiled geometry such that functionally an electric voltage would be produced in the electric channel **223C** in response to a magnetic flux change in the magnetic channel **222C** or *vice-versa.* The coiled geometry of the magnetic and electric channels, **222C** and **223C,** is generally such that at least one of the electric and magnetic channels, **223C** or **222C,** is configured to have a plurality of windings/turns (encircling) about the other one of the magnetic and electric channels, **222C** or **223C.** This thus provides that the electric channel is configured and operable as an inductor **220I** of the electric coil **220** and the magnetic channel is configured and operable as a magnetic core **220M** of the electric coil **220** to yield higher induced voltage in the coil **220** in response to magnetic fields changes and/or *vice-versa.*

As indicated above the electric component(s) **210** printed in the technique of the present invention includes at least one electric coil **220** having an open magnetic circuit configuration. The open magnetic circuit configuration facilitates efficient coupling of magnetic flux from an external magnetic field B to pass through the magnetic core **220M** of the open magnetic circuit coil **220** thereby enabling yield of high induced voltage in response to magnetic flux changes. The open magnetic circuit configuration of the magnetic channel is typically implemented such that the distal-end facets **222E₁** and **222E₂** of the magnetic channel **222C** are generally spaced apart (not connected to one another). Typically, the distal-end facets **222E₁** and **222E₂** are arranged to face different directions (e.g. opposite directions) so as to facilitate efficient flow of magnetic flux from an external magnetic field through the magnetic channel **222C.** Moreover, the spatial distribution of magnetic material **222** in the magnetic channel **222C** (between the distal-end facets **222E₁** and **222E₂** thereof) in the model is such that the conductive channel **223C** is not enclosed by a closed loop of the magnetic material **222.** More specifically the spatial distribution of magnetic material **222** of the magnetic channel **222C** does not define a continuous set of adjacent magnetic material voxels forming a closed path of the magnetic material surrounding the conductive channel **223C.**

It should be noted that in the specific non-limiting examples shown in the figures the coiled geometry of the electric and magnetic channels **223C** and **222C** of the modeled coil **220** is implemented by a helical/spiral geometry of the electric channel **223C** and with the magnetic channel **222C** configured to occupy a region at the center of the helical/spiral geometry of the electric channel **223C.** Thus, in these non-limiting examples the electric channel **223C** is configured to have a plurality of windings/turns **223W** about the magnetic channel **222C.** Nonetheless, it should be appreciated that the present application is not limited by this specific geometry, and that models presenting other coiled geometries of the magnetic and electric channels, **222C** and **223C** that functionally producing induction voltages/currents in the electric channel **223C** in response to change in magnetic flux in the magnetic channel **222C** or *vice-versa* may also be implemented/3D printed according to the technique of the invention without departing from the scope of the present Application.

In order to achieve the open magnetic circuit coil configuration effectively, the model includes a spatial distribution of another material, being a non-magnetic electrically insulating material **221** to serve as a bulk material. The electric channel **223C** between the two electric terminals **223T₁** and **223T₂** thereof, is fully embedded within the bulk material **221** such that voxels thereof interface either voxels of the bulk material **221,** voxels of the magnetic material **222,** or both, and are not located at edge facets of its respective electric components **210,** except for the two electric terminals **223T₁** and **223T₂** which may optionally be exposed at edge facet(s) of the electric components **210** to facilitate electrical connection to the coil **220.**

In this connection it should also be noted that the inductor **220I** is preferably fully embedded within the bulk **220B** of the electric component **220** such that the conductive material thereof **223** is not exposed at external surfaces of the bulk **220B** (except possibly for the two electric terminals **223T₁** and **223T₂**) and thereby isolated from environmental conditions which might degrade the physical/conductive properties of the conductive material **223** (e.g. due to excessive oxidation which might for example occur under the high temperature conditions of the sintering process; or storage in humid air). Specifically, it would be appreciated protection of the conductive material from environmental conditions is specifically important when implementing the technique of the present invention for the fabrication of miniature electric coil components. For example, the present invention facilitates fabrication of an open magnetic circuit coil **220** with conductive windings **223W** having thickness as small 5µ along the electric channel **223C** of the inductor **220I.** Such small features may easily be damaged by environmental conditions, and therefore embedding them within the bulk **220B** is particularly important in order preserve proper functionality of the coil, while allowing it to be subjected to some of the high temperature processes performed during the 3D printing without functional damage.

Turning back to **Fig. 1A****,** in operation **120** of method **100,** a multi-material 3D-printing is applied to print the one or more electric components **210** based on the model to thereby obtain the one or more electric components **210** with the at least one open magnetic circuit electric coil **220** embedded therein. The multi-material 3D-printing may be carried out for example by utilizing a photopolymerization 3D-printing technique to print layers that are composed of a combination of several materials (e.g., Two, three or more) having different electromagnetic properties. For example, 3D printing of electric components with spatial distribution of at least three materials including: electrically-conductive material, magnetic material and non-magnetic dielectric material. With the technique of the invention electric components with small feature sizes can be printed utilizing 3D printed layers with thicknesses in the scale down to microns (e.g., sintered layer thickness of 8 microns) and with pixelated (in-later) features of characteristic sizes in the scale of tens of microns or less. The printing may be implemented for example based on the principles of Vat Photopolymerization, utilizing for instance stereolithography (SLA) or digital light processing (DLP) for the pattern implementation/projections. The invention is not limited to these specific technologies and may generally be implemented by other multi-material 3D printing technique as may be developed to be suitable for the fabrication of the electric components according to the invention.

For carrying out the 3D printing, curable resins corresponding respectively to the at least three materials designated by the model are provided. More specifically the curable resins used in the 3D printing of the electronic components **210** according to the invention include at least the following:
- A magnetic material resin which includes particles of a soft magnetic material magnetic material **222** suspended in curable polymer binder. The particles of the magnetic material **222** may include for example particles of Ceramic-Ferrite material. It should be noted that alternatively in some embodiments the particles of the magnetic material **222** may include particles of magnetic Metal Materials such as Iron alloy, iron-silicon alloy, iron-aluminum-silicon alloys, low-carbon steels, nickel-iron alloys, iron-cobalt alloys, ferrites, amorphous alloys, as well as in some cases Nickel-Cobalt Alloy (particularly in cases it has soft magnetic qualities). In embodiments where miniature coils are fabricated the particles of magnetic material used may be provided for example with characteristic sizes in the micron to submicron scale in embodiments where the magnetic channel **220M** of the coil manifests delicate features.
- A conductive material resin which includes particles of conductive material **223** suspended in curable polymer binder. The particles of the conductive material **223** may for instance include Silver material, Copper material and/or Silver-Palladium alloy. As specific choice of suitable conductive material may generally depend on the required temperatures of the sintering/firing process required to finalize the printing of the electric components and may thus depend on the types of magnetic and dielectric materials. In embodiments where miniature coils are fabricated having conductive windings with characteristic sizes in the scale of several microns or few tens of microns, the particles of conductive material **223** used in the printing may have characteristic sizes in the submicron scale and as small as in the nanometer scale in cases where the printing of such small features is desired.
- A dielectric and non-magnetic material resin which includes particles of non-magnetic dielectric material **221** suspended in curable polymer binder. The particles of the non-magnetic dielectric material typically include ceramic or glass-ceramic particles which can withstand the high sintering/firing temperatures required for sintering the printed conductive and magnetic materials. Also, here the size of the particles may be selected according to the minimal feature sizes that should be occupied by the dielectric material/bulk of the electric components, and for fabrication of miniature coils may be selected for instance in the order of nanometer to submicron scale.

In regards to the material selection in the above, it should be noted that although printing magnetic channel **222C** with magnetic metal material is generally possible, in various embodiments printing the magnetic channel with an electrically insulating ceramic ferrite may be preferred. One reason for that is that the use ceramic ferrite facilitates achieving more compact arrangement of magnetic and electric channels, **222C** and **223C.** This is because soft magnetic metal materials (non-ceramic ferrite) that are suitable for use for the magnetic channel printing, are typically conductive. Therefore, a magnetic channel **222C** printed with the non-ceramic ferrite should be spaced from the electric channel **223C** (in order to prevent short circuit). This for one requires design and printing of a more complex model of the electric component in which intermediate voxels of the dielectric materials (buffer of ceramic or glass-ceramic material) should be located between the magnetic channel **222C** and the electric channel **223C** at regions where these channels may otherwise interface one another. Additionally, it may impact the achievable miniaturization of the component due to the required separation between the conductive magnetic channel **222C** and the electric channel **223C** that should preferably be of at least a few microns for sufficient electric isolation. Moreover, when
using such non-ceramic ferrite materials, and with present printing equipment, the separation is in practice larger typically in the order of 10(s) or more microns, according to the minimal lateral (in-layer) characteristic sizes supported by present printing equipment. Therefore preferably, ceramic ferrite material is used for the printing of the magnetic channel **222C**, and particularly when printing of electric components including miniature coils having high sensitivity/induced-voltages.

Thus, utilizing the at least three curable raisings described above, 3D printing of the electric components **210** may be performed layer by layer according to the three-dimensional spatial distribution of the materials in the model.

As indicated above the printing may be for example be carried out utilizing photopolymerization multilateral 3D printing techniques such as SLA or DLP. Preferably the printing direction by which the printing is conducted is substantially perpendicular to planes spanned by the conductive windings **223W.** I.e., the printing directions is preferably the Z direction illustrated in **Fig. 1B** which is perpendicular to the X-Y planes about which the windings are (approximately) spanned. With the current 3D-printing technologies, this choice of the printing direction Z may have particular importance in the printing of miniature open magnetic circuit coils **220,** in order to achieve a high-density of windings in such miniature coils (e.g., high count of conductive windings **223W** of the inductor **220I** per unit length of the magnetic core **220M).** This is because with the current 3D-printing technologies the lateral feature resolution in the X-Y plane is lower than the "vertical" resolution obtained along the printing direction z of the layers (e.g., the lateral resolution of some 3d printing technologies facilitates small feature sizes of about 25 µm and even down to 15µm while the thicknesses of the printed layers (and accordingly the "vertical" feature sizes), may be substantially smaller, below 10 µm and even down to only one or few microns (e.g. 5µm).

With reference to **Figs. 1C** and **1D****,** four successive lateral cross-sections **(a), (b), (c)** and **(d)** of a model of an electric coil **220** according to an example of the present invention are schematically illustrated in self-explanatory manner in **Fig. 1C**, as well as a vertical cross-section (e) which is schematically illustrated in self-explanatory manner in **Fig. 1D****.** The lateral cross-sections **(a), (b), (c)** and **(d)** are taken from lateral planes X-Y perpendicular to the printing direction Z and present four respective printable layers of the model. In an example the cross-sections **(a), (b), (c)** illustrate three consecutive layers of the electric coil **220.**

To this end, typically the 3D printing of each layer of the open magnetic circuit electric coil(s) **220** typically includes the following (not necessarily in that order):
- printing and curing the magnetic material **222** at regions of the layer corresponding to the magnetic core(s) **220M** of the electric coils;
- printing and curing the conductive material **223** at regions of the layer corresponding to inductor(s) **220I** of the electric coil(s); and
- printing and curing the non-electrically-conductive (dielectric) non-magnetic material **221** at least near interfaces of the conductive material **223** (possibly excluding the contact regions **223T₁** and **223T₂)** which are not at interface with the magnetic material **222** (this is in order to embed the conductive material **223** in the bulk of the electric component **210** for protection against degradation due to environmental conditions.
Thus, by the 3D printing **120,** the structures of one or more electric component(s) **210** which include at least one electric coil **220** of open magnetic circuit configuration is obtained.

In the present example, as shown by **Figs. 1B** to **1D****,** the 3D printing is carried out such that the magnetic core **220M** of the open magnetic circuit coil **220** of the occupies the central region of the coil/helical geometry of inductor **220I** while not enclosing the conductive windings of the inductor with a continuous closed loop/channel of the magnetic material **222.**

In this connection, it should be noted that in some embodiments of the present invention the method **100** is carried out for 3D-printing electric components **110** in which miniature electric coils having features of micron scale size are embedded. For instance, the 3D printed layers may be printed with thicknesses in a range of about 5 to 15 µm (e.g., 9µm), depending on the specific model design and 3D-Printing technology used. Accordingly, a pitch of the coiled/helical arrangement of windings **223W,** which may be in the order of twice the pitch of the 3D printed layers, may be within the range of about 10µm to 30µm along the printing direction **Z** of the 3D printed layers (e.g., winding pitch of about 18 µm).

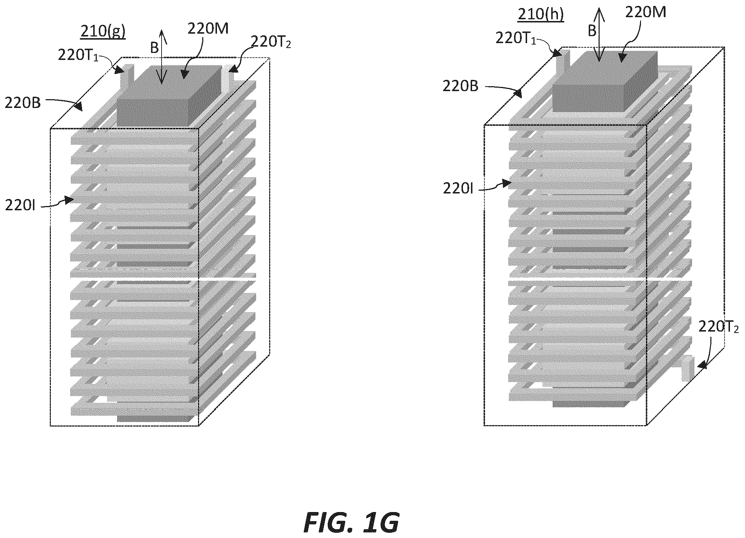

It should be understood that although in the above described non limiting examples the coil windings **223W** are distributed at different 3D printed layers of the electric component **210** and have helical configuration, other winding configurations may be implemented in the open magnetic circuit electric coils that are fabricated by the technique of the present invention without departing from the scope of the present Application. For instance, a plurality of windings may be arranged in each of one or more of the 3D printed layers of the electric component **210** and may be connected in a spiral geometry within such layers or in form of a concentric double-/multi- helix configuration across multiple layers as illustrated for instance in **Fig. 1G****.** Accordingly open magnetic circuit coils with windings **223W** of a single-helix geometry may be fabricated and/or in order to facilitate higher density of the windings **223W** within a miniature coil, the windings **223W** may be configured and fabricated by the technique of the invention with a concentric multi-helix geometry and/or with a spiral-helical geometry. For instance, in a spiral-helical arrangement of windings, a one or more spiral windings may be distributed at different one or more 3D printed layers within the **220B** bulk and connected between them to form the spiral-helical arrangement. Alternatively, or additionally, in a double-/multi-helix geometries of the windings, concentric helixes (or spiral-helixes) of windings which may be connected in parallel or series to one another, may be fabricated within the open magnetic circuit coil **220.**

In such embodiments where concentric windings **223W** or parts thereof are fabricated within the same 3D printed layer, the concentric parts are typically spaced by the 3D printed non-magnetic dielectric material **221** in order to preserve the open magnetic circuit geometry of the coil **220** while also not shortcutting the windings' path. In some embodiments the 3D printing is performed with lateral printing resolution of the 3D printed layers in the order of 15 to 30 µm (typically about 25 µm). In this regard it would be appreciated that the projector typically projects pixel with resolution between 19 µm to 35µm and during the sintering of the printed components their dimensions shrink by about 20% thus yielding in layer resolution/feature-sizes between 15 to 30 µm.

Accordingly, in some implementations of the invention where miniature coil **220** is fabricated with inclusion of concentric in-layer windings **223W** (e.g., with multi-helix or spiral-helix geometries), a lateral pitch of in-layer windings may be in the order of about 30µm to 60µm (e.g., typically about 50µm), being about twice the lateral resolution of the 3D printing. This may thereby be implemented to further improve the spatial densities of the windings in the miniature coils and yield improvement in the voltage induced in response to varying magnetic fields.

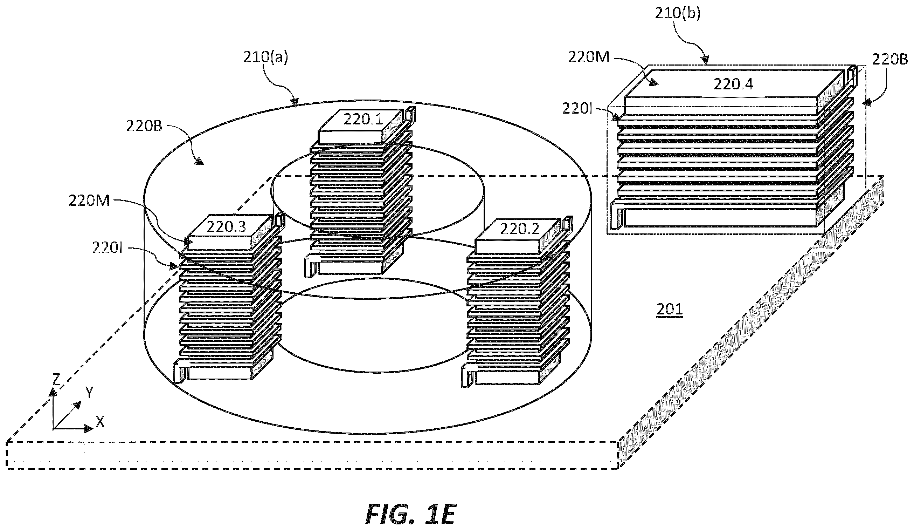

With reference to **Fig. 1E****,** two examples of electric components **210(a)** and **210(b)** are shown on a printing platform **201,** after being printed by operations **110** and **120** of the method **100** of the invention. The two electric components exemplified here are made suitable for use as sensors for sensing the location and/or orientation of medical instrument such as a catheter (e.g., relative to an external frame of reference) - component **210(b),** and for sensing the force applied by the medical instrument/catheter to a tissue in contact therewith - component **210(a).** In this particular nonlimiting example the electric component **210(a)** has a ceramic or glass-ceramic bulk **220B** formed with a ring/donut shape with three miniature open magnetic circuit coils **220.1, 220.2** and **220.3** of substantially similar properties distributed over the circumference of the ring/donut shapes bulk **220B** and co-aligned with their magnetic axes along a symmetry axis **Z** of the donut shape's bulk. Accordingly, this electric component may be fitted within a catheter's elongated body with its symmetry axis **Z** aligned to the longitudinal axis of the catheter's body and is suitable for sensing its orientation relative to a magnetic field source placed in front of it along the longitudinal axis at a distal part of the catheter (thus may serve for sensing a force applied by the catheter to a tissue). In this particular nonlimiting example, the component **210(b)** is a single open magnetic circuit coil with small height along the **Z** direction of its magnetic axis and having elongated lateral aspect ratio over the **X-Y** plane, making it suitable for fitting within a catheter's elongated body for sensing a component of a magnetic field perpendicular to the longitudinal axis Z of the catheters elongated body with sufficient sensitivity. Accordingly, two such components **210(b)** arranged in the catheter with different directions of their magnetic axes perpendicular to the catheters longitude, together with one of the coils of the component **210(a)** whose magnetic axis is aligned with the catheters longitude, may be used to sense the position and orientation of the catheter relative to a source of external magnetic field residing externally from the catheter.

Turning back to **Fig. 1A****,** method **100** further includes operation **130** for sintering the one or more 3D structures forming the electric components **210** printed in the 3D-printing operation **120.** The sintering is generally performed at temperature sufficient for burning or driving off polymers (binding polymers) from the printed electric components **210** as well as firing ceramic or glass ceramic materials used in the printing. To this end, after the sintering the material density of the printed ceramic and metal materials (e.g., **221, 222** and **223)** of the electric components **210** approaches 100%. For example, in embodiments where the conductive material **223** mainly includes Silver, the sintering may be performed at temperatures below the silver melting point (e.g., at temperatures not exceeding 961°C (preferable in this case the sintering temperature may be limited up to about 900C in order to achieve a stable sintering process). In other embodiments where the conductive material includes a Silver-Palladium alloy, the sintering may be performed at temperatures below the alloy melting point (the specific melting point depends on the ratio of silver and palladium in the alloy and depending on the composition the melting point is generally between the melting point of silver, 961°C, and that of palladium, 1555°C).

In another example, in embodiments where the conductive material **223** includes Copper, the sintering may be performed in an Oxygen deprived environment (so as to avoid oxidation of the Copper), and at temperatures below the 1084°C Copper melting point.

Further, optionally (depending on the specific printing technology used), the method **100** may include operation **140** for separating the electric components **210** from the building platform (e.g., **201** shown in **Fig. 1E****).** Generally, depending on the type of building platform **201** (e.g., and whether it can withstand the sintering **130),** the operation **140** is typically performed before the sintering **130.** Indeed, in some embodiments of the invention, particularly where miniature/delicate electric components are printed, which may be damaged by carrying out their separation from the building platform before the sintering, a suitable building **201** platform may be used which can withstand the temperatures of the sintering **130,** and the components separation from the platform may be conducted post sintering so as to avoid/reduce damage to the components during the separation process.

Further, optionally, in some embodiments it is also desired to furnish one or more of the 3D printed electric components **210** with surface mounts for their installation on a circuit board via surface mount technology (SMT). According to some embodiments of the invention, surface mounts suitable for the SMT are 3D-printed together with the 3D-printing of the components **210.** In such embodiments typically materials such as silver-palladium alloy are used for the SMT mounts' electric contacts, since the conventional materials, Tin and Nickel-Tin which are typically used in SMT mounts' electric contacts, are not suitable for the sintering process of the 3D printing technology of the invention (Tin's melting point is too low and Nickel-Tin will oxidize in the process). To this end in some embodiments the 3D-printing operation **120** of method **100** further includes 3D printing of the external/SMT contacts. The 3D printed SMT contacts may be for example fabricated from silver-palladium alloy (Ag-Pd) and formed with thickness of about 9µm to yield external Ag-Pd SMT electrodes for the 3D printed electric components **210.**

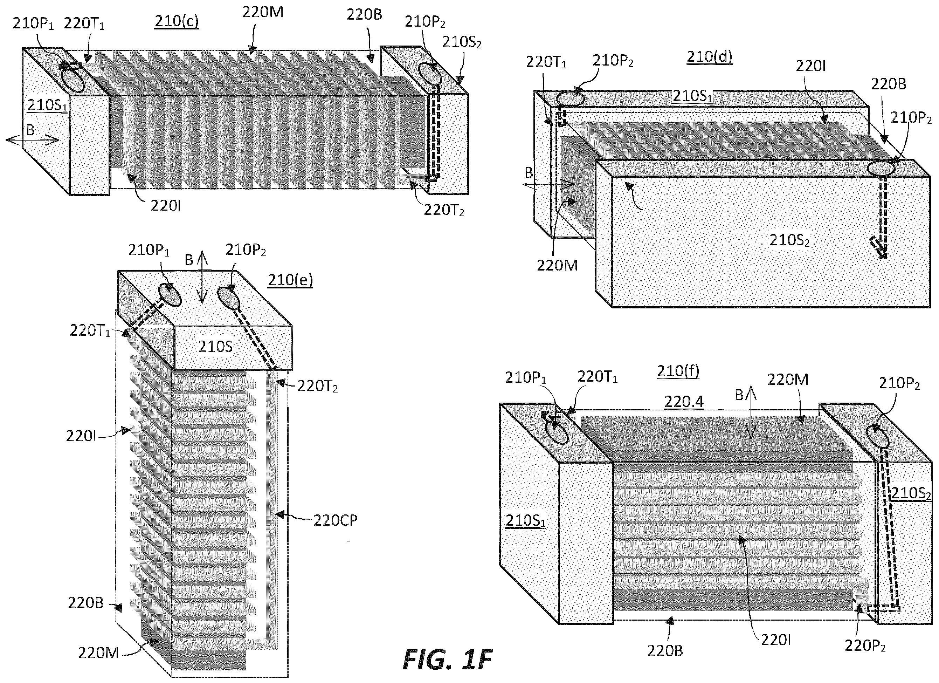

Alternatively, or additionally, in some embodiments of the invention the SMT contacts/mounts, or some of them, are fabricated/attached to the 3D printed electric components **210** only after the 3D-printing and Sintering of the components **210.** To this end, in some embodiments method **100** further includes operation **150** for fabricating/attaching SMT surface mounts to the 3D printed electric components **210.** With reference to **Fig. 1F**, four electric components **210(c), 210(d), 210(e)** and **210(f)** each including a single open magnetic circuit coil 3D-printed by the technique of the present invention are illustrated in self-explanatory manner. The four electric components **210(c), 210(d), 210(e)** and **210(f),** are shown with different variations of surface mounts **210S, 210S₁** and **210S₂** coupled and electrically connected to the 3D-printed components. In this example, the surface mounts **210S, 210S₁** and **210S₂** arranged at each coil components, include a pair of electric contacts/pads (e.g., made of Tin or Nickel+Tin) **210P₁** and **210P₂** which are electrically connected to the respective electric terminals **220T₁** and **220TP₂** of the respective coils **220** of the components and are suitable for soldering to a circuit board via SMD technology. As shown in the figure, in the two variations of the electric components **210(c)** and **210(d),** the SMT mounts are furnished such that the coils **220** will be mounted to a circuit board with a so-called horizontal orientation, such that their magnetic axis **B** is parallel to a surface of the circuit board. The components **210(c)** and **210(d)** differ by the respective arrangement of the SMT mounts where in **210(c)** the mounts are arranged along the magnetic axis B (to yield a more narrow and elongated dimensions of the component), and in **210(d)** the mounts are arranged aside/parallel to the magnetic axis B are **210(d)** (to yield shorter and wider dimensions of the component). In the two variations of the electric components **210(e)** and **210(f),** the SMT mounts are furnished such that the coils **220** will be mounted to a circuit board with a so-called vertical orientation, such that their magnetic axis **B** is perpendicular to a surface of the circuit board. The components **210(e)** and **210(f)** differ by the respective arrangement of the SMT mounts where in **210(e)** a single mount is arranged along the magnetic axis B (to yield a narrower and more elongated/taller dimensions of the component), and in **210(f)** the mounts are arranged aside/parallel to the magnetic axis B are **210(d)** (to yield shorted and wider dimensions of the component). Accordingly, as shown in the figure the present invention facilitates different arrangements of the SMT mounts on the 3D-printed electric components to yield desired optimization of the total dimensions of the components with the mounts to fit desired real-estate space allocated thereto inside a device, such as a catheter in which there are installed, as well as facilitating proper selection of the magnetic axis orientation of the components relative to the circuit board - to facilitate their proper accommodation for sensory application in which the available real-estate for their placement is in shortage. Notably, it should be understood that the fabrication of the SMT mounts **210S, 210S₁** and **210S₂** may be carried out according to any suitable technique known in the art (e.g., by electroless or electrochemical deposition of for example Nickel (Ni) and/or Tin (Sn)), or during the 3D-Printing in **120** as indicated above. Also notably, that in embodiments where the SMT mounts **210S, 210S₁** and **210S₂** are arranged to face the magnetic axis/es of the coil(s), their main body is preferably made of non-magnetically-permeable material so as not to screen magnetic flux from the coil.

With reference together to **Figs. 1E** to **1G****,** several variations of electric components **210(a)** to **210(g)** with 3D-printed open magnetic coils according to the present invention are illustrated. As shown in the coils of all of those electric components **210(a)** to **210(g),** the boundaries of the bulk **220B** of the electric components/coils are printed with one or more materials other than the conductive material **223** so as to protect the conductive material of the inductor **220I** from damage due to environmental conditions. In other words, the conductive material of the inductor **220I** is fully embedded within a bulk **220B** of its respective electronic component and not exposed at external surfaces of the bulk except **220B** from electric contacts thereof. As indicated above, spacings between adjacent conductive windings may be occupied by 3D printed non-electrically-conductive material (e.g., the dielectric bulk material **221,** or the magnetic material **222** in case it is not conductive). For example, as shown for instance in components **210(c)** and **210(d)** the conductive windings of the inductor **220I** may be spaced from one another by the magnetic material **223** of the magnetic core **220M**, (which is an insulator in these examples). This is as long as the magnetic material **223** does not form a closed loop over the conductive windings, and thus preserve the open magnetic circuit configuration of the coil. Moreover, in some embodiments the magnetic material may extend in places to the boundary of the electric component, as shown for instance in the component **210(c).** In other embodiments, e.g. component **210(e),** the windings may be spaced by the dielectric bulk material **221.** As shown in the component **210(e),** in some embodiments the model of the electric component may also include a model for extending a conductive path **220CP** from the inductor **220I** of the coil to a terminal electric contact thereof, e.g., **220T₂** or to another component located any place in the bulk **220B.** Furthermore, as indicated above, the inductor **220I** may be fabricated as with a coaxial/multi-helical configuration, as shown for example in the double helix of the component **210(g),** and/or with spiral-helical configuration as shown for example in the component **210(h).**

To this end the technique of the present invention provides miniature electric components having a ceramic/glass-ceramic body embedding therein highly sensitive open magnetic circuit coils embedded which may be configured and operable as sensors (e.g., magnetic sensors and/or position sensors and/or orientation sensors and/or force sensors). The miniature electric components may be fabricated with small dimensions, for example in the order of ~0.5 mm to ~2 mm and with high sensitivity/induced-voltage in response to magnetic fields, for example in the range ~0.1 to 1 µV/(Gauss*Hz) corresponding to 0.001 to 0.01 V/(T*Hz))

It should be noted that the illustrations of the electrical components in **Figs. 1B** to **1G****,** are provided for illustrative purposes only, and are not necessarily drawn to scale or with proper proportions. For example, generally the count of winding in open circuit electric coils **220** according to the present invention may be substantially higher than what is illustrated in the figure. Also, open circuit electric coils **220** are illustrated with rectangular shapes/cross-sections, whereby it should be understood that in practice they may be 3D-printed with various polygonal or rounded shapes/cross-sections, and or with different aspect ratios than those illustrated, without departing from the scope of the present invention, which is defined by the appended claims.

In view of the above, method **100** of the present invention provides a novel and inventive technique for fabrication of electric components including open magnetic circuit coils via 3D printings. The method **100** is suited for fabrication of miniature coils with features sizes as small as few microns, and may be used in mass production (particularly when the printing is based on vat photopolymerization technology), in order to simultaneously 3D print large pluralities (e.g., typically between hundreds and several thousand electric components built in parallel on the same platform) of miniature coils in one printing batch. This makes the technique of the present invention cost effective for printing miniature coils which typically otherwise require one-by-one fabrication while typically requiring manual fabrication steps, when fabricated by the conventional techniques.

To this end the present invention also provides novel and inventive electric component formed by 3D printing of at least three different 3D-printed materials and including one or more electric coils of open circuit configuration. The electric components as exemplified for instance by **Figs. 1E to 1G** include a bulk formed by non-magnetic and electrically insulating 3D printed material with at least one electric coil in the bulk including: (a) at least one magnetic channel **222C** formed by 3D-printed magnetic material embedded in the bulk; and (b) at least one electric channel **223C** formed by 3D-printed conductive material embedded in the bulk and extending therethrough between two terminals which may be exposed at a surface of the bulk to serve as electric contacts for the electric coil. At least one of the electric or magnetic channels is 3D printed with coiled geometry to form a plurality of windings about the other one such that the electric channel functions as an inductor of the coil and the magnetic channel as a magnetic core thereof (for example the electric channel may be 3D-printed with a coil/helical geometry having a plurality of windings about the magnetic channel). The 3D printed magnetic channel which forms the magnetic core is configured as an open magnetic circuit configuration such that it does not enclose the conductive channel with a closed loop of magnetic material.

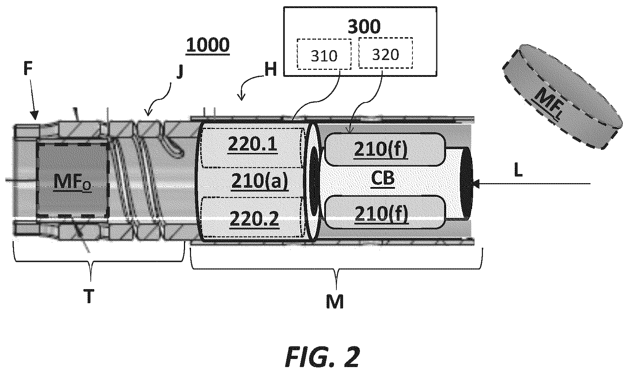

As indicated above the 3D printed electric components **210** of the present invention with the open magnetic circuit coils **220** therein are particularly suited for use as position and/or force sensors in medical instrument requiring the same. Reference is made to **Fig. 2** illustrating a side-view of a medical instrument **1000** (e.g., probe/catheter) according to an embodiment of the present invention in which position and force sensors utilizing the 3D printed electric components **210** of the present invention with the open magnetic sensor coils therein for sensing the spatial location and orientation of the medical instrument as well as the force applied thereby to a tissue in contact therewith. The medical instrument **1000** includes a housing **H** with a magnetic field-based position sensor **300** furnished therein. The medical instrument **1000** is associated with one or more magnetic field sources **MF_{L}** and/or **MF_{O}** which provide reference magnetic fields which can be measured by the magnetic field-based position sensor **300** to respectively determine the spatial location and orientation of the medical instrument **1000** and/or the force applied thereby to a patient's tissue.

In an embodiment where force applied by the medical instrument **1000** to a body tissue, the medical instrument's housing **H** may be arranged along longitudinal axis **L** and include a main section **M** and a tip section **T** coupled at a distal end of the main section **M** via a banding coupler **J.** The banding coupler **J,** which may be for example a spring or joint, is configured such that the orientation of the tip section **T** can be bent relative to the longitudinal axis **L** of the housing **H** under applied force **F** (which may be applied for example when tip section **T** of the medical instrument is in contact with a body tissue of a patient) with banding degree corresponding to a magnitude and direction of the applied force **F.** In order to measure the magnitude and direction of a force **F** applied by the tip **T** to a tissue, the medical instrument **1000** includes a first magnetic field source **MF_{O}** (e.g., magnetic field generating coil with associated power delivery circuitry) and a magnetic-field-based positioning sensor **300.** The orientation sensor **310** and the first magnetic field source **MF_{O}** are arranged in the housing at different sides of the joint **J,** for instance typically the first magnetic field source **MF_{O}** is arranged in the tip section **T** and the orientation sensor **310** at the main section **M** (although opposite arrangement may also be applicable). The first magnetic field source **MF_{O}** is configured and arranged in the housing **H** (e.g. in the tip **T)** for producing a magnetic field with flux lines/axis generally along the longitudinal axis **L** of the housing **H.** The orientation sensor **310** includes a plurality of at least three open magnetic circuit coils **220.1, 220.2** and **220.3** according to the present invention which are capable of sensing the magnetic field generated by the first magnetic field source **MF_{O}**. The three open magnetic circuit coils **220.1, 220.2** and **220.3** of the orientation sensor **310** are generally arranged with their magnetic axes collinear to one another and parallel to the longitudinal axis **L** of the housing **H** while not being co-planar, so that a relative orientation between the orientation sensor **310** and the magnetic field source **MF_{O}** can be determined about two orientation axes (e.g. pitch and yaw) by a magnitude of the magnetic field for the source **MF_{O}** sensed by the three coils, as will be appreciated by those versed in the art. In the present nonlimiting example, the housing has a narrow width/diameter of about 2 mm, and the three open magnetic circuit coils **220.1, 220.2** and **220.3** are parts of the electric component **210(a)** illustrated in **Fig. 1E****,** which is fabricated according to the technique of the present invention. As indicated above, the electric component **210(a)** which has solid ceramic or glass-ceramic bulk/body is fitted in the main section **M** of the housing and optionally connected directly to signal lines (not specifically shown) for receiving the coils signals without a need for an intermediate circuit board (the signal lines may be soldered directly to the terminals of the coils **220.1, 220.2** and **220.3** in the electric component **210(a)).** Preferably the ceramic or glass-ceramic body of the electric component **210(a)** has a cylindrical/donut structure as shown above with a hole in the middle and is fitted with its symmetry axis parallel to the longitudinal axis of the housing such that various parts/connection lines of the instrument can be arranged to pass therethrough from the main section of the housing to the tip section (for instance passage of power delivery lines to the magnetic field source and/or other connection/fluid lines which may be furnished in the medical instrument). Advantageously, utilizing the configuration of the electric component **210(a)** for the orientation sensor **310** facilitates reliable, accurate and cost-effective fitting and electrical connection of the three open magnetic circuit coils **220.1, 220.2** and **220.3** within the narrow dimensions of the housing **H.** It should be noted that in alternative embodiment the orientation sensor **310** may include the three open magnetic circuit coils **220.1, 220.2** and **220.3** as separate electric components fabricated according to the present invention. For instance, the orientation sensor **310** may include a rigid flat circuit board (e.g., disk like shaped having a hole in the middle) with three electronic component such as **210(e)** described above with reference to **Fig. 1F** furnished vertically on the rigid rounded circuit board by their SMT contacts such that the open magnetic circuit coils embedded therein serve as the coils **220.1, 220.2** and **220.3** the orientation sensor **310.** In yet another embodiment the orientation sensor **310** may be implemented with a flexible/foil circuit board, such as the flex board **CB** of the location sensor **320** discussed below, which is flexed about the longitudinal axis **L** of the housing **H** to a cylindrical shape. Three electronic component such as **210(c)** described above with reference to **Fig. 1F** may be furnished horizontally on cylindrical flexed board (e.g., **CB)** by their SMT contacts such that their magnetic axes are parallel to the longitudinal axis **L** thus providing that the open magnetic circuit coils embedded therein serve as the coils **220.1, 220.2** and **220.3** the orientation sensor **310.** To this end, having considered the technique of the present invention person of ordinary skill in the art will readily appreciate how the orientation of the sensor **310** relative to the magnetic field source **MF_{O}** may be determined based on measurements of the magnetic field generated by the source **MF_{O}**. Moreover, a person of ordinary skill in the art will readily appreciate how the force vector **F** applied to the tissue may be determined based on the measured orientation and the properties (e.g., spring constant) of the banding coupler **J.**

Alternatively, or additionally, in some embodiments the position sensor **300** includes a location & orientation sensor **320** that is adapted to measure the spatial location and orientation of the medical instrument **1000** relative to a frame of reference provided by magnetic fields from at least one external magnetic field source **MF_{L}** (located externally to the medical instrument). The location & orientation sensor **320** includes three open magnetic circuit coils **220** configured according to the technique of the present invention and arranged in the housing **H** such that their magnetic axes are not parallel to one another and span three dimensional coordinates. Accordingly, the three open magnetic circuit coils **220** facilitate measurement of different vector components of the magnetic field generated by the external magnetic field source **MF_{L}**, and by such measurement, as will be readily be appreciated by those versed in the art, a location and orientation of the medical instrument **1000** can be determined relative to the reference frame defined by the magnetic field of the external magnetic field source **MF_{L}**.

In embodiments, as for instance illustrated in the figure, the location & orientation sensor **320** includes a flexible circuit board **CB** folded to a cylindrical shape, with at least two preferably similar open magnetic circuit coils **220** according to the present invention, furnished on the flexible circuit board **CB** such that their magnetic axes are substantially not parallel to one another and also not parallel (typically perpendicular) to the longitudinal axis **L** of the housing **H.** The two preferably similar open magnetic circuit coils **220** may be for example coils implemented by electronic components such as **210(b)** of the present invention which have high magnetic sensitivity while with low profile/height along their magnetic axes so that they can be fitted within the narrow housing with their magnetic axes perpendicular to the longitudinal axis **L** of the housing **H.** Preferably, as shown in the figure, the two open magnetic circuit coils **220** may be implemented by electronic components such as **210(f)** of the present invention, which are similar to **210(b)** only having SMT contacts fitted thereon. Accordingly, the electronic components **210(f)** with the two coils thereof can be furnished on the flexible board **CB** by cost effective SMT mounting technology. In addition, the location & orientation sensor **320** includes a third open magnetic circuit coil arranged in the housing such that its magnetic axis is not parallel to the magnetic axes of the two other coils, and preferably such that its magnetic axis is substantially parallel to the longitudinal axis **L** of the housing **H.** In embodiments as illustrated in the figure, where the position sensor **300** includes also the orientation sensor **310,** the third open magnetic circuit coil of the location & orientation sensor **320** may be one of the coils of the orientation sensor **310,** for example coil **220.1.** Alternatively, for instance in embodiments where the position sensor **300** does not include an orientation sensor **310,** the third open magnetic circuit coil may also be arranged on the flexible board **CB** (e.g., with its axis substantially parallel to the longitudinal axis **L).** In such embodiments the third coil may be implemented by an electric component of the invention, for example by electric component **210(c)** illustrated in **Fig. 1F****,** and may optionally be furnished on the flexible board **CB** via SMT technology.

Thus, the present invention provides novel and inventive position sensor **300** configuration, utilizing novel and inventive 3D-printed open magnetic circuit coils to facilitate cost effective and reliable fitting of the position within medical instruments of narrow dimensions. As indicated above the position sensor **300** may include an orientation sensor **310** suitable for example for determining a force applied by the medical instrument to a tissue and including three open magnetic circuit coils configured according to the present invention, and/or a location & orientation sensor **320** including three open magnetic circuit coils configured according to the present invention and operable to determine the spatial location and orientation of the medical instrument **1000** (in cases where the position sensor **300** includes both sensors **310** and **320**, a total of five open magnetic circuit coils may be sufficient for carrying out the measurements).

## Claims

1. An electric component (210) for use in a position sensor of a catheter, comprising one or more electric coils (220), the electric component is formed by 3D printing of at least three different 3D-printed materials with spatial distributions yielding an open magnetic circuit configuration of at least one electric coil of said one or more electric coils (220), wherein the 3D printing comprises providing a respective curable resin for each of the at least three different 3D-printed materials; and wherein the electric component (210) comprises:
a bulk (220B) formed by non-magnetic and dielectric 3D printed material (221); and
at least one electric coil (220) of the open magnetic circuit configuration, 3D-printed in said bulk (220B), comprising:
- at least one magnetic channel (222C) comprising magnetic material (222) 3D-printed in said bulk (220B) forming a magnetic core (220M) of the at least one electric coil (220); and
- at least one electric channel (223C) forming an inductor (220I) of said at least one electric coil (220) comprising conductive material (222) 3D-printed in said bulk (220B) with a coil/helical geometry having a plurality of electrically connected conductive windings (223W) arranged to circumference the magnetic channel (222C) of the magnetic core (220M);
wherein the magnetic channel (222C) of the magnetic core (220M) is configured and operable with said open magnetic circuit configuration and comprises magnetic material (222) occupying a central region of the coil/helical geometry of the inductor (220I) while not enclosing said conductive windings (223W) with a closed loop of said magnetic material (222).

2. The electric component (210) of claim 1 wherein said electrically connected conductive windings (223W) comprise conductive windings distributed at different 3D printed layers of said electric component (210) thereby forming a helical arrangement of electrically connected conductive windings.

3. The electric component (210) of claim 2 wherein a thickness of said 3D printed layers is in the order of 5 to 15 µm thereby yielding a pitch of said helical arrangement of conductive windings (223W) in the order of twice the pitch of said 3D printed layers being about 10µm to 30µm along a printing direction of said 3D printed layers.

4. The electric component (210) of any one of claims 1 to 3 wherein said electrically connected conductive windings (223W) comprise plurality of conductive windings arranged concentrically in one or more 3D printed layers of said electric component thereby forming a spiral arrangement of conductive windings in said the one or more 3D printed layers.

5. The electric component (210) of claim 4 wherein a resolution of said 3D printed layers, after said sintering, is in the order of 15 to 30 µm, thereby yielding a pitch of said spiral arrangement of conductive windings in the order of twice the resolution of said 3D printed layers with respect to a lateral plane of said 3D printed layers; and preferably wherein said electrically connected conductive windings comprise a plurality of said spiral arrangement of conductive windings distributed at different 3D printed layers of said bulk and electrically connected between them to form a spiral-helical arrangement of said electrically connected conductive windings.

6. The electric component (210) of any one of claims 1 to 5, wherein spacings between adjacent windings of said a plurality of electrically connected conductive windings (223W) are occupied by 3D printed non-electrically-conductive material being one of said non-magnetic dielectric material (221) and said 3D-printed magnetic material (222).

7. The electric component (210) of any one of claims 1 to 6, wherein the 3D-printed conductive material (223) of the inductor (220I) is fully embedded within said bulk (220B) and not exposed at external surfaces of said bulk (220B) , so as to isolate the inductor (220I) from environmental conditions which might degrade its physical/conductive properties.

8. A method (100) to fabricate one or more electric components (210) for use in a position sensor of a catheter, comprising one or more electric coils (220);
wherein at least one electric coil of said one or more electric coils (220) has an open magnetic circuit configuration; the method comprising:
providing a model (110) of the one or more electric components (210) wherein the model is indicative of three-dimensional spatial distribution of at least three materials in the at least one electric coil (220) having the open magnetic circuit configuration, including: 3D-distribution of magnetic material (222), 3D-distribution of conductive material (223), and 3D-distribution of non-magnetic dielectric material (221);
wherein the three-dimensional spatial distribution of the at least three materials is indicative of:
- spatial distribution of a bulk (210B) of the at least one electric coil formed by non-magnetic dielectric material (221);
- spatial distribution of magnetic material (222), defining at least one magnetic channel (222C) in said bulk (210B) associated with a magnetic core (220M) of the at least one electric coil (220);
- spatial distribution of conductive material (223) defining at least one electric channel in said bulk (210B) associated with an inductor (220I) of said at least one electric coil (220) and having a coiled/helical geometry with a plurality of electrically connected conductive windings (223W) arranged to circumference said magnetic channel (222C);
wherein the spatial distribution of the magnetic material (222) has said open magnetic circuit configuration and is indicative of magnetic material (222) occupying a central region of the coil/helical geometry of said inductor (220I) while not enclosing the conductive windings (223W) of said inductor (220I) with a closed loop of said magnetic material; and
applying 3D printing (120) to print said one or more electric components thereby obtaining said at least one electric coil with the open magnetic circuit configuration facilitating efficient coupling of flux passage from an external magnetic field through the magnetic core to yield high induced voltage in response to the external magnetic field; wherein applying the 3D printing comprises providing curable resins corresponding respectively to the at least three materials.

9. The method of claim 8 wherein said curable resins comprise:
- magnetic material resin comprising particles of magnetic material (222) suspended in curable polymer binder;
- conductive material resin comprising particles of conductive material (223) suspended in curable polymer binder;
- non-magnetic dielectric material resin comprising particles of non-magnetic dielectric material (221) suspended in curable polymer binder; and wherein said applying of the 3D printing (120) comprises:
**(a)** 3D printing said curable resins layer by layer according to said three-dimensional spatial distribution of the model to obtain one or more 3D structures with said one or more electric components (210), wherein 3D printing each layer comprises:
- printing and curing said magnetic material (222) at regions of said layer corresponding to one or more magnetic cores (220M) of said one or more electric coils;
- printing and curing said conductive material (223) at regions of said layer corresponding to one or more inductors (220I) of said one or more electric coils (220); and
- printing and curing said of non-magnetic dielectric material (221) at least near interfaces of said conductive material (223) of the one or more inductors not interfacing said one or more magnetic cores (220M); and
wherein said 3D printing carried out such that the magnetic core (220M) of said at least one coil (220) of the open magnetic circuit configuration occupies the central region of the coil/helical geometry of said inductor (220I) while not enclosing the conductive windings (223W) of said inductor (220I) with a continuous closed loop channel of said magnetic material (222); and
**(b)** sintering (130) said one or more 3D structures at temperature sufficient for burning or driving off polymers therefrom and thereby achieving ceramic or metal density approaching 100% in said electric components (210).

10. The method of Claim 8 or 9 wherein said 3D printing is carried out with successive printing of layers of thicknesses in the order of 5 to 15 µm along a printing direction (z) and such that a pitch of the conductive windings along said printing direction (z) is in the order twice said layer thicknesses of about 10µm to 30µm thereby yielding printing of miniature electric coils having high winding density.

11. The method of any one of Claims 8 to 10 suitable for mass-production of electronic components and wherein said model is indicative of material distribution in an arrangement of a plurality of said electric components (210) to be simultaneously 3D printed.

12. The method of any one of Claims 8 to 11 wherein said 3D printing is carried out utilizing vat photopolymerization 3D printing process.

13. The method of any one of Claim 8 to 12 wherein at least one of the following:
- said conductive material comprises Silver, and the method comprises sintering said one or more 3D structures at temperatures below 961°C;
- said conductive material comprises Copper, and the method comprises sintering said one or more 3D structures in Oxygen deprived environment (such as Nitrogen rich environment) at temperatures below 1084°C;
- said conductive material comprises Silver-Palladium alloy, and the method comprises sintering said one or more 3D structures at temperatures below a melting point of the Silver-Palladium alloy;
- said magnetic material comprises Ceramic-Ferrite material;
- said magnetic material comprises Metal material;
- said magnetic material has a relative permeability µᵣ in the order of 100 or more;
- said non-magnetic dielectric material comprises Ceramic or Glass-Ceramic material and wherein the method comprises sintering said one or more 3D structures at temperature sufficient for firing said Ceramic or Glass-Ceramic material.

14. The electric component of claim 1, wherein the electric component (210) is a magnetic sensor (300).

15. The method (100) of claim 8, wherein the one or more electric components (210) are one or more magnetic sensors (300).

## Patentansprüche

1. Elektrische Komponente (210) zur Verwendung in einem Positionssensor eines Katheters, umfassend eine oder mehrere elektrische Spulen (220), wobei die elektrische Komponente durch 3D-Druck von mindestens drei unterschiedlichen 3D-gedruckten Materialien mit räumlichen Verteilungen ausgebildet wird, die eine offene magnetische Schaltungskonfiguration von mindestens einer elektrischen Spule der einen oder der mehreren elektrischen Spulen (220) ergeben, wobei der 3D-Druck ein Bereitstellen eines jeweiligen härtbaren Harzes für jedes der mindestens drei unterschiedlichen 3D-gedruckten Materialien umfasst; und wobei die elektrische Komponente (210) umfasst:
eine Masse (220B), die aus nicht magnetischem und dielektrischem 3D-gedrucktem Material (221) ausgebildet ist; und
mindestens eine elektrische Spule (220) der offenen magnetischen Schaltungskonfiguration, die in dem Volumenkörper (220B) 3D-gedruckt ist, umfassend:
- mindestens einen magnetischen Kanal (222C), umfassend magnetisches Material (222), das in der Masse (220B) 3D-gedruckt ist, wobei ein Magnetkern (220M) der mindestens einen elektrischen Spule (220) ausgebildet wird; und
- mindestens einen elektrischen Kanal (223C), der einen Induktor (2201) der mindestens einen elektrischen Spule (220) ausbildet, umfassend leitfähiges Material (222), das in der Masse (220B) 3D-gedruckt ist, mit einer Spulen-/Wendelgeometrie, die eine Vielzahl von elektrisch verbundenen leitfähigen Windungen (223W) aufweist, die angeordnet sind, um den magnetischen Kanal (222C) des Magnetkerns (220M) zu umgeben;
wobei der magnetische Kanal (222C) des Magnetkerns (220M) mit der offenen magnetischen Schaltungskonfiguration konfiguriert und betreibbar ist und magnetisches Material (222) umfasst, das einen zentralen Bereich der Spulen-/Wendelgeometrie des Induktors (2201) belegt, während es die leitfähigen Windungen (223W) nicht mit einer geschlossenen Schleife des magnetischen Materials (222) umschließt.

2. Elektrische Komponente (210) nach Anspruch 1, wobei die elektrisch verbundenen leitfähigen Windungen (223W) leitfähige Windungen umfassen, die auf unterschiedliche 3D-gedruckte Schichten der elektrischen Komponente (210) verteilt sind, wodurch eine spiralförmige Anordnung elektrisch verbundener leitfähiger Windungen ausgebildet wird.

3. Elektrische Komponente (210) nach Anspruch 2, wobei eine Dicke der 3D-gedruckten Schichten in der Größenordnung von 5 bis 15 µm liegt, wodurch eine Steigung der spiralförmigen Anordnung von leitfähigen Windungen (223W) in der Größenordnung des Doppelten der Steigung der 3D-gedruckten Schichten von etwa 10 µm bis 30 µm entlang einer Druckrichtung der 3D-gedruckten Schichten erreicht wird.

4. Elektrische Komponente (210) nach einem der Ansprüche 1 bis 3, wobei die elektrisch verbundenen leitfähigen Windungen (223W) eine Vielzahl von leitfähigen Windungen umfassen, die konzentrisch in einer oder mehreren 3D-gedruckten Schichten der elektrischen Komponente angeordnet sind, wodurch eine spiralförmige Anordnung von leitfähigen Windungen in der einen oder den mehreren 3D-gedruckten Schichten ausgebildet wird.

5. Elektrische Komponente (210) nach Anspruch 4, wobei eine Auflösung der 3D-gedruckten Schichten nach dem Sintern in der Größenordnung von 15 bis 30 µm liegt, wodurch eine Steigung der spiralförmigen Anordnung leitfähiger Windungen in der Größenordnung des Doppelten der Auflösung der besagten 3D-gedruckten Schichten bezüglich einer lateralen Ebene der 3D-gedruckten Schichten erreicht wird; und vorzugsweise wobei die elektrisch verbundenen leitfähigen Windungen eine Vielzahl der spiralförmigen Anordnung von leitfähigen Windungen umfassen, die an unterschiedlichen 3D-gedruckten Schichten der Masse verteilt und zwischen ihnen elektrisch verbunden sind, um eine spiralhelixförmige Anordnung der elektrisch verbundenen leitfähigen Windungen auszubilden.

6. Elektrische Komponente (210) nach einem der Ansprüche 1 bis 5, wobei Abstände zwischen angrenzenden Windungen der Vielzahl von elektrisch verbundenen leitfähigen Windungen (223W) durch 3D-gedrucktes nicht elektrisch leitfähiges Material belegt sind, das eines von dem nicht magnetischen dielektrischen Material (221) und dem 3D-gedruckten magnetischen Material (222) ist.

7. Elektrische Komponente (210) nach einem der Ansprüche 1 bis 6, wobei das 3D-gedruckte leitfähige Material (223) des Induktors (2201) vollständig innerhalb der Masse (220B) eingebettet und an äußeren Oberflächen der Masse (220B) nicht freigelegt ist, um die Induktoren (2201) von Umgebungsbedingungen zu isolieren, die ihre physikalischen/leitfähigen Eigenschaften verschlechtern könnten.

8. Verfahren (100) zum Herstellen einer oder mehrerer elektrischer Komponenten (210) zur Verwendung in einem Positionssensor eines Katheters, umfassend eine oder mehrere elektrische Spulen (220);
wobei mindestens eine elektrische Spule der einen oder der mehreren elektrischen Spulen (220) eine offene magnetische Schaltungskonfiguration aufweist; wobei das Verfahren umfasst:
Bereitstellen eines Modells (110) der einen oder der mehreren elektrischen Komponenten (210), wobei das Modell eine dreidimensionale räumliche Verteilung von mindestens drei Materialien in der mindestens einen elektrischen Spule (220) anzeigt, die die offene magnetische Schaltungskonfiguration aufweist, einschließlich: 3D-Verteilung von magnetischem Material (222), 3D-Verteilung von leitfähigem Material (223) und 3D-Verteilung von nicht magnetischem dielektrischem Material (221);
wobei die dreidimensionale räumliche Verteilung der mindestens drei Materialien anzeigt:
- räumliche Verteilung einer Masse (210B) der mindestens einen elektrischen Spule, die aus nicht magnetischem dielektrischem Material (221) ausgebildet ist;
- räumliche Verteilung von magnetischem Material (222), das mindestens einen magnetischen Kanal (222C) in der Masse (210B) definiert, die einem Magnetkern (220M) der mindestens einen elektrischen Spule (220) zugeordnet ist;
- räumliche Verteilung von leitfähigem Material (223), das mindestens einen elektrischen Kanal in der Masse (210B) definiert, die einem Induktor (2201) der mindestens einen elektrischen Spule (220) zugeordnet ist und eine spulenförmige/Wendel-Geometrie mit einer Vielzahl von elektrisch verbundenen leitfähigen Windungen (223W) aufweist, die angeordnet sind, um den magnetischen Kanal (222C) zu umgeben;
wobei die räumliche Verteilung des magnetischen Materials (222) die offene magnetische Schaltungskonfiguration aufweist und anzeigt, dass das magnetische Material (222) einen zentralen Bereich der Spulen-/Wendelgeometrie der Induktors (2201) einnimmt, während es die leitfähigen Windungen (223W) des Induktors (2201) nicht mit einer geschlossenen Schleife des magnetischen Materials umschließt; und
Anwenden von 3D-Druck (120), um die eine oder die mehreren elektrischen Komponenten zu drucken, wodurch die mindestens eine elektrische Spule mit der offenen magnetischen Schaltungskonfiguration erhalten wird, die eine effiziente Kopplung eines Flussdurchgangs von einem externen Magnetfeld durch den Magnetkern ermöglicht, um eine hohe induzierte Spannung als Reaktion auf das externe Magnetfeld zu ergeben; wobei das Anwenden des 3D-Drucks das Bereitstellen härtbarer Harze umfasst, die jeweils den mindestens drei Materialien entsprechen.

9. Verfahren nach Anspruch 8, wobei die härtbaren Harze umfassen:
- Magnetmaterialharz, umfassend Partikel aus magnetischem Material (222), die in härtbarem Polymerbindemittel suspendiert sind;
- leitfähiges Materialharz, umfassend Partikel aus leitfähigem Material (223), die in härtbarem Polymerbindemittel suspendiert sind;
- nicht magnetisches dielektrisches Materialharz, umfassend Partikel aus nicht magnetischem dielektrischem Material (221), die in härtbarem Polymerbindemittel suspendiert sind; und wobei das Anwenden des 3D-Drucks (120) umfasst:
(a) 3D-Druck der härtbaren Harze Schicht für Schicht gemäß der dreidimensionalen räumlichen Verteilung des Modells, um eine oder mehrere 3D-Strukturen mit der einen oder den mehreren elektrischen Komponenten (210) zu erhalten, wobei der 3D-Druck jeder Schicht umfasst:
- Drucken und Aushärten des magnetischen Materials (222) an Bereichen der Schicht, die einem oder mehreren Magnetkernen (220M) der einen oder der mehreren elektrischen Spulen entsprechen;
- Drucken und Aushärten des leitfähigen Materials (223) an Bereichen der Schicht, die einem oder mehreren Induktoren (2201) der einen oder der mehreren elektrischen Spulen (220) entsprechen; und
- Drucken und Aushärten des nicht magnetischen dielektrischen Materials (221) mindestens nahe Grenzflächen des leitfähigen Materials (223) des einen oder der mehreren Induktoren, die nicht mit dem einen oder den mehreren Magnetkernen (220M) in Kontakt stehen; und
wobei der 3D-Druck derart ausgeführt wird, dass der Magnetkern (220M) der mindestens einen Spule (220) der offenen magnetischen Schaltungskonfiguration den zentralen Bereich der Spulen-/Wendelgeometrie des Induktors (2201) belegt, während er die leitfähigen Windungen (223W) des Induktors (2201) nicht mit einem kontinuierlichen geschlossenen Schleifenkanal des magnetischen Materials (222) umschließt; und
(b) Sintern (130) der einen oder der mehreren 3D-Strukturen bei einer Temperatur, die ausreichend ist zum Abbrennen oder Austreiben von Polymeren daraus und dadurch Erreichen einer Keramik- oder Metalldichte von annähernd 100 % in den elektrischen Komponenten (210).

10. Verfahren nach Anspruch 8 oder 9, wobei der 3D-Druck mit sukzessivem Drucken von Schichten mit Dicken in der Größenordnung von 5 bis 15 µm entlang einer Druckrichtung (z) durchgeführt wird und derart, dass eine Steigung der leitfähigen Windungen entlang der Druckrichtung (z) in der Größenordnung des Doppelten der Schichtdicken von etwa 10 µm bis 30 µm liegt, wodurch das Drucken von elektrischen Miniaturspulen erreicht wird, die eine hohe Windungsdichte aufweisen.

11. Verfahren nach einem der Ansprüche 8 bis 10, geeignet für eine Massenproduktion von elektronischen Komponenten und wobei das Modell eine Materialverteilung in einer Anordnung einer Vielzahl der elektronischen Komponenten (210) anzeigt, die gleichzeitig 3D-gedruckt werden sollen.

12. Verfahren nach einem der Ansprüche 8 bis 11, wobei der 3D-Druck unter Verwendung eines Wannen-Photopolymerisations-3D-Druck-Prozesses ausgeführt wird.

13. Verfahren nach einem der Ansprüche 8 bis 12, wobei mindestens eines der Folgenden:
- das leitfähige Material Silber umfasst, und das Verfahren das Sintern der einen oder der mehreren 3D-Strukturen bei Temperaturen unter 961 °C umfasst;
- das leitfähige Material Kupfer umfasst, und das Verfahren das Sintern der einen oder der mehreren 3D-Strukturen in einer sauerstoffarmen Umgebung (wie einer stickstoffreichen Umgebung) bei Temperaturen unter 1084 °C umfasst;
- das leitfähige Material eine Silber-Palladium-Legierung umfasst, und das Verfahren das Sintern der einen oder der mehreren 3D-Strukturen bei Temperaturen unterhalb eines Schmelzpunkts der Silber-Palladium-Legierung umfasst;
- das magnetische Material Keramik-Ferrit-Material umfasst;
- das magnetische Material Metallmaterial umfasst;
- das magnetisches Material eine relative Permeabilität µᵣ in der Größenordnung von 100 oder mehr aufweist;
- das nicht magnetische dielektrische Material Keramik- oder Glaskeramikmaterial umfasst und wobei das Verfahren das Sintern der einen oder der mehreren 3D-Strukturen bei einer Temperatur umfasst, die zum Ausbrennen des Keramik- oder Glaskeramikmaterials ausreicht.

14. Elektrische Komponente nach Anspruch 1, wobei die elektrische Komponente (210) ein Magnetsensor (300) ist.

15. Verfahren (100) nach Anspruch 8, wobei die eine oder die mehreren elektrischen Komponenten (210) ein oder mehrere Magnetsensoren (300) sind.

## Revendications

1. Composant électrique (210) destiné à être utilisé dans un capteur de position d'un cathéter, comprenant une ou plusieurs bobines électriques (220), le composant électrique étant formé par impression 3D d'au moins trois matériaux différents imprimés en 3D avec des répartitions spatiales produisant une configuration de circuit magnétique ouvert d'au moins une bobine électrique de ladite ou desdites bobines électriques (220),dans lequel l'impression 3D comprend la fourniture d'une résine durcissable respective pour chacun des au moins trois matériaux différents imprimés en 3D ; et dans lequel le composant électrique (210) comprend :
une masse (220B) formée par un matériau imprimé en 3D non magnétique et diélectrique (221) ; et
au moins une bobine électrique (220) de la configuration de circuit magnétique ouvert, imprimée en 3D dans ladite masse (220B), comprenant :
- au moins un canal magnétique (222C) comprenant un matériau magnétique (222) imprimé en 3D dans ladite masse (220B) formant un noyau magnétique (220M) de l'au moins une bobine électrique (220) ; et
- au moins un canal électrique (223C) formant un inducteur (2201) de ladite au moins une bobine électrique (220) comprenant un matériau conducteu (222) imprimé en 3D dans ladite masse (220B) avec une géométrie de bobine/hélicoïdale ayant une pluralité d'enroulements conducteurs (223W) connectés électriquement et agencés de manière à entourer le canal magnétique (222C) du noyau magnétique (220M) ;
dans lequel le canal magnétique (222C) du noyau magnétique (220M) est configuré et utilisable avec ladite configuration de circuit magnétique ouvert et comprend un matériau magnétique (222) occupant une région centrale de la géométrie de bobine/hélicoïdale de l'inducteur (2201) sans enfermer lesdits enroulements conducteurs (223W) avec une boucle fermée dudit matériau magnétique (222).

2. Composant électrique (210) selon la revendication 1, dans lequel lesdits enroulements conducteurs (223W) connectés électriquement comprennent des enroulements conducteurs répartis au niveau de différentes couches imprimées en 3D dudit composant électrique (210), formant ainsi un agencement hélicoïdal d'enroulements conducteurs connectés électriquement.

3. Composant électrique (210) selon la revendication 2, dans lequel une épaisseur desdites couches imprimées en 3D est de l'ordre de 5 à 15 µm, produisant ainsi un pas dudit agencement hélicoïdal d'enroulements conducteurs (223W) de l'ordre de deux fois le pas desdites couches imprimées en 3D, soit environ 10 µm à 30 µm le long d'une direction d'impression desdites couches imprimées en 3D.

4. Composant électrique (210) selon l'une quelconque des revendications 1 à 3, dans lequel lesdits enroulements conducteurs (223W) connectés électriquement comprennent une pluralité d'enroulements conducteurs agencés de manière concentrique dans une ou plusieurs couches imprimées en 3D dudit composant électrique, formant ainsi un agencement en spirale d'enroulements conducteurs dans ladite ou lesdites couches imprimées en 3D.

5. Composant électrique (210) selon la revendication 4, dans lequel une résolution desdites couches imprimées en 3D, après ledit frittage, est de l'ordre de 15 à 30 µm, produisant ainsi un pas dudit agencement en spirale d'enroulements conducteurs de l'ordre de deux fois la résolution desdites couches imprimées en 3D par rapport à un plan latéral desdites couches imprimées en 3D ; et, de préférence, dans lequel lesdits enroulements conducteurs connectés électriquement comprennent une pluralité dudit agencement en spirale d'enroulements conducteurs répartis au niveau de différentes couches imprimées en 3D de ladite masse et connectés électriquement entre eux pour former un agencement en spirale hélicoïdale desdits enroulements conducteurs connectés électriquement.

6. Composant électrique (210) selon l'une quelconque des revendications 1 à 5, dans lequel des espacements entre des enroulements adjacents de ladite une pluralité d'enroulements conducteurs (223W) connectés électriquement sont occupés par un matériau non électriquement conducteur imprimé en 3D, qui est l'un dudit matériau diélectrique non magnétique (221) et dudit matériau magnétique imprimé en 3D (222).

7. Composant électrique (210) selon l'une quelconque des revendications 1 à 6, dans lequel le matériau conducteur imprimé en 3D (223) de l'inducteur (2201) est entièrement intégré à l'intérieur de ladite masse (220B) et n'est pas exposé au niveau de surfaces externes de ladite masse (220B), de manière à isoler l'inducteur (2201) des conditions environnementales qui pourraient dégrader ses propriétés physiques/conductrices.

8. Procédé (100) permettant de fabriquer un ou plusieurs composants électriques (210) destinés à être utilisés dans un capteur de position d'un cathéter, comprenant une ou plusieurs bobines électriques (220) ;
dans lequel au moins une bobine électrique de ladite ou desdites bobines électriques (220) a une configuration de circuit magnétique ouvert ; le procédé comprenant :
la fourniture d'un modèle (110) du ou des composants électriques (210), dans lequel le modèle indique une répartition spatiale tridimensionnelle d'au moins trois matériaux dans l'au moins une bobine électrique (220) ayant la configuration de circuit magnétique ouvert, comportant : la répartition 3D de matériau magnétique (222), la répartition 3D de matériau conducteur (223) et la répartition 3D de matériau diélectrique non magnétique (221) ;
dans lequel la répartition spatiale tridimensionnelle des au moins trois matériaux indique :
- la répartition spatiale d'une masse (210B) de l'au moins une bobine électrique formée d'un matériau diélectrique non magnétique (221) ;
- la répartition spatiale de matériau magnétique (222), définissant au moins un canal magnétique (222C) dans ladite masse (210B) associée à un noyau magnétique (220M) de l'au moins une bobine électrique (220) ;
- la répartition spatiale de matériau conducteur (223) définissant au moins un canal électrique dans ladite masse (210B) associée à un inducteur (2201) de ladite au moins une bobine électrique (220) et ayant une géométrie en bobine/hélicoïdale avec une pluralité d'enroulements conducteurs (223W) connectés électriquement agencés de manière à entourer ledit canal magnétique (222C) ;
dans lequel la répartition spatiale du matériau magnétique (222) a ladite configuration de circuit magnétique ouvert et indique qu'un matériau magnétique (222) occupe une région centrale de la géométrie de bobine/hélicoïdale dudit inducteur (2201) sans enfermer les enroulements conducteurs (223W) dudit inducteur (2201) avec une boucle fermée dudit matériau magnétique ; et
l'application d'une impression 3D (120) pour imprimer ledit ou lesdits composants électriques, obtenant ainsi ladite au moins une bobine électrique avec une configuration de circuit magnétique ouvert facilitant un couplage efficace de passage de flux d'un champ magnétique externe à travers le noyau magnétique afin de produire une tension induite élevée en réponse au champ magnétique externe ; dans lequel l'application d'une impression 3D comprend la fourniture de résines durcissables correspondant respectivement aux au moins trois matériaux.

9. Procédé selon la revendication 8, dans lequel lesdites résines durcissables comprennent :
- une résine de matériau magnétique comprenant des particules de matériau magnétique (222) en suspension dans un liant polymère durcissable ;
- une résine de matériau conducteur comprenant des particules de matériau conducteur (223) en suspension dans un liant polymère durcissable ;
- une résine de matériau diélectrique non magnétique comprenant des particules de matériau diélectrique non magnétique (221) en suspension dans un liant polymère durcissable ; et dans lequel ladite application de l'impression 3D (120) comprend :
(a) l'impression 3D desdites résines durcissables couche par couche selon ladite répartition spatiale tridimensionnelle du modèle pour obtenir une ou plusieurs structures 3D avec ledit ou lesdits composants électriques (210), dans lequel l'impression 3D de chaque couche comprend :
- l'impression et le durcissement dudit matériau magnétique (222) au niveau de régions de ladite couche correspondant à un ou plusieurs noyaux magnétiques (220M) de ladite ou desdites bobines électriques ;
- l'impression et le durcissement dudit matériau conducteur (223) au niveau de régions de ladite couche correspondant à un ou plusieurs inducteurs (2201) de ladite ou desdites bobines électriques (220) ; et
- l'impression et le durcissement dudit matériau diélectrique non magnétique (221) au moins à proximité d'interfaces dudit matériau conducteur (223) du ou des inducteurs n'interférant pas avec ledit ou lesdits noyaux magnétiques (220M) ; et
dans lequel ladite impression 3D est réalisée de telle sorte que le noyau magnétique (220M) de ladite au moins une bobine (220) de la configuration de circuit magnétique ouvert occupe la région centrale de la géométrie de bobine/hélicoïdale dudit inducteur (2201) sans enfermer les enroulements conducteurs (223W) dudit inducteur (2201) avec un canal en boucle fermée continu dudit matériau magnétique (222) ; et
(b) le frittage (130) de ladite ou desdites structures 3D à une température suffisante pour brûler ou chasser des polymères qu'elles contiennent et obtenir ainsi une densité céramique ou métallique proche de 100 % dans lesdits composants électriques (210).

10. Procédé selon la revendication 8 ou 9, dans lequel ladite impression 3D est réalisée par impression successive de couches d'épaisseurs de l'ordre de 5 à 15 µm le long d'une direction d'impression (z) et de telle sorte qu'un pas des enroulements conducteurs le long de ladite direction d'impression (z) est de l'ordre de deux fois lesdites épaisseurs de couche d'environ 10 µm à 30 µm, produisant ainsi une impression de bobines électriques miniatures ayant une densité d'enroulement élevée.

11. Procédé selon l'une quelconque des revendications 8 à 10, adapté à une production de masse de composants électroniques et dans lequel ledit modèle indique une répartition de matériau dans un agencement d'une pluralité de composants électriques (210) à imprimer simultanément en 3D.

12. Procédé selon l'une quelconque des revendications 8 à 11, dans lequel ladite impression 3D est réalisée à l'aide d'un procédé d'impression 3D par photo polymérisation en cuve.

13. Procédé selon l'une quelconque des revendications 8 à 12, dans lequel au moins l'un parmi les éléments suivants :
- ledit matériau conducteur comprend de l'argent, et le procédé comprend le frittage de ladite ou desdites structures 3D à des températures inférieures à 961 °C ;
- ledit matériau conducteur comprend du cuivre, et le procédé comprend le frittage de ladite ou desdites structures 3D dans un environnement privé d'oxygène (tel qu'un environnement riche en azote) à des températures inférieures à 1 084 °C ;
- ledit matériau conducteur comprend un alliage argent-palladium, et le procédé comprend le frittage de ladite ou desdites structures 3D à des températures inférieures au point de fusion de l'alliage argent-palladium ;
- ledit matériau magnétique comprend un matériau céramique-ferrite ;
- ledit matériau magnétique comprend un matériau métallique ;
- ledit matériau magnétique a une perméabilité relative µᵣ de l'ordre de 100 ou plus ;
- ledit matériau diélectrique non magnétique comprend un matériau céramique ou vitrocéramique et dans lequel le procédé comprend le frittage de ladite ou desdites structures 3D à une température suffisante pour cuire ledit matériau céramique ou vitrocéramique.

14. Composant électrique selon la revendication 1, dans lequel le composant électrique (210) est un capteur magnétique (300).

15. Procédé (100) selon la revendication 8, dans lequel le ou les composants électriques (210) sont un ou plusieurs capteurs magnétiques (300).
